# EUROPEAN PATENT SPECIFICATION

(11) **EP 1 238 679 B1**
(45) Date of publication and mention of the grant of the patent: **25.01.2006**
(21) Application number: 02425121.7
(22) Date of filing: 04.03.2002
(51) Int. Cl.: A61L 9/013, A61L 9/014, B01D 53/06, B01D 53/34, B01J 8/18

(54) **A process of deodorizing and damping evil-smelling organic materials from fumes and/or gases**
Prozess zur Deodorisierung und Geruchsdämpfung von schlecht riechenden organischen Substanzen in Dämpfen und/oder Gasen
Procédé de désodorisation et d'atténuation des matériaux organiques d'odeur désagréable contenus dans des vapeurs et/ou des gaz

(30) Priority: 06.03.2001 IT FR010005
(43) Date of publication of application: 11.09.2002
(73) Proprietor: ENEA - ENTE PER LE NUOVE TECNOLOGIE, L'ENERGIA E L'AMBIENTE, 00196 Roma (IT); Mazzanti, Uranio, 01017 Tuscania (Viterbo) (IT)
(72) Inventor: Mazzanti, Uranio, 01017 Tuscania (Viterbo) (IT)
(74) Representative: Sarpi, Maurizio

(56) References cited:
- DE-A- 10 030 958
- GB-A- 1 472 896
- GB-A- 1 578 865
- US-A- 4 292 285

## Description

### Field of the Invention

The present invention relates to a process of deodorizing and damping evil-smelling organic materials from fumes and/or gases providing the inlet and the intimate dispersion of absorbing and/or reactive solid particles which are able to provide a supporting surface for condensation, deposition and the next separation of the materials to be removed into the gaseous medium to be deodorized.

The decomposition or the separation of the collected materials from the absorbing powder particles is carried out in a second step of preferably thermal regeneration of the powder particles before their recycle or partial disposal.

### State of Art

The deodorization of fumes and more generally the damping of small amounts of undesired generally organic materials, when the latter come from incomplete combustion, is generally carried out by a reheating process. This is made by means of a specific apparatus in which fumes are brought to high temperature by inlet of generally gaseous fuel (natural gas) and oxygen in the presence of suitable catalyzers.

Even energetic cost of such system is often prohibitive considering that a great amount of air with low thermal coefficient should be brought to high temperature without any possibility of regenerating the produced energy. Therefore, this solution is used in great industrial installations and when the gaseous medium to be deodorized has a large enthalpy or heat content before the reheating process.

When the materials to be removed are present in a gaseous medium which has not enough high temperature to allow its reheating, other systems such as filtration on biologically active materials (biofilters) or an usual treatment with liquids in scrubbers are effectively used.

Scrubbers are almost used especially employing water as liquid which is the medium in which the substances extracted from the gaseous stream are flowing.

Depuration of water or water solution of a specific reagent is a problem involving high cost which cannot easily be solved.

In the field of pollution of the olfactory sensation the thresholds of evil-smell tolerance are being studied. In Italy there is no specific standard or limit values for the evil-smell emissions. ANPA (National Agency for the Protection of Environment) has launched a smell measurement campaign by means of human and electronic "noses". The sensorial methods, however, do not allow single chemical elements to be detected but only permit a reliable opinion on the olfactory nuisance to be given.

Evil smells (but not toxic emissions) do not harm or damage the health seriously until the olfactory detection of a certain concentration thereof and the stay time of the subject in contact therewith do not exceed the limit of the tolerance threshold. However, such evil smells irritate and sometimes are unbearable causing side effects such as difficulty of concentration, state of depression, sick feeling and retching Well-known examples are rubbish damps, cattle-breeding but also coffee roasting and biscuit factories insofar as the nuisance comes not only from inherently unpleasant smells but also from pleasant smells with high intensity and/or continuous emission. Sensational examples are fast foods and the already known "smog burger" effect associated thereto. A research carried out by California University showed that restaurants and fast foods of the district of Los Angeles give out 19 tons of organic materials per day, i.e. about 1.5 tons more than the major oil refineries in the same area.

According to US-A-4292285, GB-A-1472896 and GB-A-1578865 are disclosed processes for removing compounds having offensive odor from gases containing the same, using solid particles made of selected substances pretreated and activated in order to present suitable properties.

### Scope of the Invention

The process of the present invention seeks to damp the unburned materials present in the baking fumes and responsible for the above-mentioned "smog burger" effect and/or a variety of similar products also with tarry origin and tendency to polymerization which are often in the form of minute particles dispersed in large amount of gases so as to make their damping by the known washing system ineffective and their reheating unfavourable.

Such aim has been obtained by a process according to the appended claims.

### Description of the process

The main feature of the process consists of using solid particles with a large range of sizes and characteristics related to types and sizes of the polluting materials dispersed in the gaseous medium.

The solid particles fed and dispersed into the gas stream are inert but almost provided with a high capability of adsorption and absorption. In any case such particles should be able to provide a supporting surface for condensation, deposition and the next separation of the materials to be removed.

The particles that have captured the materials present in the gas are regenerated and reused in the capturing process. The regeneration can be carried out according to the kind of the employed dusts and the polluting materials to be removed (the latter being conventionally called tars from now on) by conventional single operations such as washing with solvents, roasting or chemical or biological treatment. It should be mentioned that the denomination "tars" refers, in addition to the smelling organic materials present in the vapour phase, also to all products similar to tar such as liquids and semiliquids having more or less the tendency to condensation and polymerization, including deliberately also the distillation products of fat materials from food cooking processes in such definition as well as all materials present in gaseous fuels from pyrolysis and/or thermal gasification of wastes and residual fuels, fumes of coffee roasting, mists from drying and roasting processes of other materials such as vegetable and animal flours.

According to a peculiar feature of the invention, the amount of solid particles fed and intimately dispersed into the gas stream is adjusted so that such solid particles are not completely wet by the condensing liquids and tar.

By such measure, the solid particles will be easily recovered from the gas as solid mass in the form of dry dust by means of cyclones, sleeve filt.ers, or electrostatic filters, i.e. apparatuses typically employed for the even very extreme dust exhaustion of gases that cannot instead be used when the materials dispersed in the gas to be purified are wet or viscous or fat, thus adhering to the dust exhaustion systems and making the latter inapplicable.

The solid particles that can be used in the process of the present invention can be of the most varied types, of organic or inorganic nature. In particular residual ashes from combustion processes are used as such ashes are able to provide a large supporting surface for the condensation, the deposition, and the next separation of aromatic substances such as pitch in the gas stream produced by the gasification of biomasses.

The decomposition of the materials collected with the dusts is then carried out in the thermal regeneration step of the dusts before their recycle (if such a regeneration system can be applied) or their partial disposal.

The dusts employed in the process can then be divided into two "families": disposable dusts and reusable dusts. In order to limit the financial burden, the former are used once exhausted as energy carrier. This is the case for instance of the wood flours, spent olive residues, fuels in the form of fine particles that are not provided directly for the heat regeneration but are used as energy carriers once performed their function as fume purifying means. Of course, this is the case only in particular industrial processes.

The latter family of reusable dusts is typically exemplified by mineral dusts fully or partially formed by carbonates of alkaline and alkaline-earth metals. After the first thermal regeneration these metals take the form of oxides (according to the temperature of the regeneration step) capable of reacting chemically with the polluting materials to be captured. For this purpose also ashes from biomass combustion processes can be used.

The recarbonation, which is possible in practice, produces carbonates that in turn are transformed to oxides again during the regeneration. The alkaline-earth oxides, typically lime, are materials that can easily be found, have a low cost and such features as to standardize the processes to get optimum operative conditions of the plants as far as high withstand of the dust capturing sleeve filters, regeneration times and temperatures of the exhausted oxides is concerned.

Other chemically inert dusts may be clay and diatomaceous earth: the former are effective in particular because of its fine granulometry that must be ensured; the latter because of its specific capability of capturing polluting materials within its structure. In order to improve the capture efficiency and where the financial means allows it, mixtures of both types of dusts can be used. The organic dusts also give a contribute to the heat content of the process during regeneration. Their possible incomplete combustion providing any mixture formed of oxides and carbon materials to be recycled to the capture step allows the polluting materials to be captured with high efficiency.

The thermal regeneration is carried out in a reactor in which the dusts are brought to such a high temperature as to cause the thermal cracking of the captured tars and then their combustion under controlled conditions.

The advantage of using such system over a "reheating process" is connected to the energetic efficiency. In fact, in order to ensure the thermal cracking and the next combustion of even minimum amounts of evil-smelling materials dispersed in the fumes by acting directly thereon, a very high energy consumption (often in the form of gaseous fuel in the reheating steps) is needed to bring the whole mass of the gaseous medium to the temperature at which the thermal cracking of the evil-smelling materials takes place for the time necessary to develop degradation processes effectively. According to the present invention only the limited amount of dusts that have "captured" the materials to be thermally cracked are brought to high temperature with the minimum energy consumption and the maximum efficiency under control.

In any case but in particular when the dusts to be treated include a combustible fraction in addition to the captured organic materials, the thermal regeneration system should be provided with devices able to ensure the oxidation of the volatile fraction that are given off from the regeneration oven so that the complete degradation is ensured before leaving to the atmosphere. Typically, such devices are Bunsen torches fed by gaseous fuel and provided with air excess and temperature control means and acting as catalytic aid to the combustion.

After checking of the humidity content and, if necessary, after passing through a plenum chamber (above all in case of non-thermal regeneration), the outlet dusts from the regeneration system (i.e. an oven in case the regeneration is thermal) are conveyed to the fumes to be purified by pneumatic means ensuring high dispersion and facilitating the intimate contact with the gaseous polluting medium to be purified.

Small/middle size systems let the regenerated dust stream dispersed in air into a fume stream to be treated having the same direction of flow. Such systems provides a suitable calculation of the contact time between the two streams and the turbulence to put effectively in contact polluting materials and purifying dusts. Known systems such as baffles or static stream mixers with high efficiency are possible and their application depends on the gaseous medium flow rate and channel dirt problems, with the result of a need of maintenance and a specific design according to the circumstances.

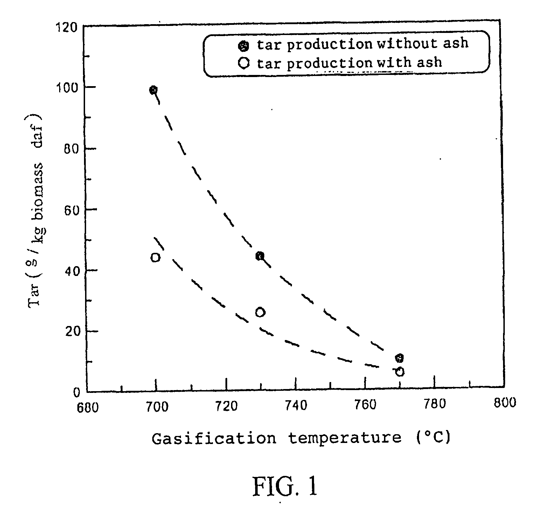

Experimental tests carried out in a standard biomass (hazel-nut shell) gasification fluid-bed installation with inlet of dusts (ashes) in the same stream direction of the gas flow, although without enhancing the turbulence, showed that the presence of ashes reduces the content of aromatic substances, "tar" (pitch), in the gas by 50% under the same temperature. In addition, it should advantageously be noted that the content of pitch is strongly decreased even at low temperatures, thus making the excessive (expensive) increase in the temperature of the gas mass useless. The result is shown in the diagram of Fig. 1.

Industrial applications can provide the contact between dusts and gaseous medium to be purified by means of a fixed or boiling or turbulence or entrainment bed through already known industrial equipment for the neutralization of acid mists, for example by sodium bicarbonate, in spray drier towers.

In the specific case of combustible gas depuration to be made uniform as far as the content of organic tarry materials is concerned, the reactive-dust inlet device varies according to the pressure conditions of the vessel in which the gas to be treated flows. If the latter is under depression, the inlet device can take advantage of the Venturi system with the care of using a gas for the dispersion of dusts which is compatible with the gas within the vessel. In order to ensure the compatibility, a fraction of the same gas can be deviated so as to act as dust fluidization carrier.

## Claims

1. A process of deodorizing and damping evil-smelling organic materials and tars from fumes and/or gases wherein solid particles are dispersed into gaseous medium to be purified, **characterized in that** the solid particles are made of inert dusts; the amount if said solid particles being adjusted so that the solid particles are not completely wet by the condensing liquid and tar.

2. The process of claim 1, **characterized in that** the inert dusts are chosen from the group consisting of: wood flours, spent olive residues, fuels in the form of fine particles, carbonate of alkaline or alkaline-earth metals, combustion residual ashes, clay and diatomaceus earth.

3. The process of claim 1, **characterized in that** the solid particles are dispersed in the gas in the same or in the opposite direction of the stream or are entrained in the gas flow by the scrubbing of the gas in dust bed.

4. The process of claim 1, **characterized in that** the dispersion of inert dusts is obtained by static flow mixers with high efficiency.

5. The process of claim 1, **characterized in that** the solid particles can be separated from the gas as solid masses in the form of dry dust by known dust exhaustion systems such as cyclones and/or sleeve filters and/or electrostatic filters.

6. The process of claim 1, **characterized in that** after the separation of the polluting medium from the gas, the solid particles are thermally regenerated to be used again in a following purification cycle by roasting in a regeneration oven.

7. The process of the preceding claim, **characterized in that** there are provided devices capable of ensuring the oxidation of the volatile fractions which are given off from the oven where the regeneration takes place so as to ensure the complete degradation before their leaving to the atmosphere.

8. The process of the preceding claim, **characterized in that** said devices are typically Bunsen torches fed by gaseous fuel and provided with air excess and temperature control means and catalytic aid to the combustion.

## Patentansprüche

1. Verfahren zum Desodorieren und Dämpfen von schlecht riechenden organischen Materialien und Teeren aus Rauch und/oder Gasen, wobei Festkörperteilchen in einem zu reinigenden gasartigen Medium verteilt werden, **gekennzeichnet dadurch, dass** die Festkörperteilchen aus inerten Stäuben bestehen, wobei die Menge der Festkörperteilchen derart angepasst ist, dass die Festkörperteilchen nicht vollständig durch die kondensierende Flüssigkeit und den Teer benetzt werden.

2. Verfahren nach Anspruch 1, **gekennzeichnet dadurch, dass** die inerten Stäube aus einer Gruppe ausgewählt sind, welche aufweist: Holzmehl, verbrauchte Olivenrückstände, Brennstoffe in Form feiner Partikel, Alkali- und Erdalkalimetallcarbonate, Verbrennungsaschrückstände, Kalk und Kieselgur.

3. Verfahren nach Anspruch 1, **gekennzeichnet dadurch, dass** die Festkörperteilchen in dem Gas in dieselbe oder in die entgegengesetzte Richtung des Stromes verteilt werden oder in dem Gasfluss durch das Waschen des Gases in dem Staubbett mitgerissen wird.

4. Verfahren nach Anspruch 1, **gekennzeichnet dadurch, dass** die Verteilung des inerten Staubes durch statische Flussmixer mit hoher Effizienz erhalten wird.

5. Verfahren nach Anspruch 1, **gekennzeichnet dadurch, dass** die Festkörperpartikel von dem Gas als feste Masse in der Form von trockenem Staub durch bekannte Staubsaugsysteme separiert werden kann, wie z. B. Zyklone und/oder Hülsenfiltern und/oder elektrostatische Filter.

6. Verfahren nach Anspruch 1, **gekennzeichnet dadurch, dass** nach der Trennung des verschmutzenden Mediums von dem Gas die Festkörperpartikel durch Rösten in einem Regenerierungsofen thermisch regeneriert werden, um wieder in einem nachfolgenden Reinigungszyklus verwendet werden zu können.

7. Verfahren nach dem vorhergehenden Anspruch, **gekennzeichnet dadurch, dass** Vorrichtungen bereitgestellt werden, welche zum Sichern der Oxidation der flüchtigen Bestandteile in der Lage sind, welche aus dem Ofen abgegeben werden, in welchem die Regenerierung stattfindet, so dass der vollständige Abbau gesichert ist, bevor sie in die Atmosphäre entweichen.

8. Verfahren nach dem vorhergehenden Anspruch, **gekennzeichnet dadurch, dass** die Vorrichtungen typischerweise Bunsenbrenner sind, welche mit einem gasförmigen Brennstoff gespeist werden und mit Luftüberschuss und einer Temperatursteuerungseinrichtung und katalytischen Hilfe für die Verbrennung ausgestattet sind.

## Revendications

1. Procédé de désodorisation et d'atténuation de matières organiques nauséabondes et de brais, issus de fumée et/ou de gaz dans lesquels des particules solides sont dispersées dans un milieu gazeux, pour être purifiés, **caractérisé en ce que** les particules solides sont constituées de poussières inertes ; la quantité desdites particules solides étant ajustée de sorte que les particules solides ne soient pas complètement mouillées par le liquide de condensation et les brais.

2. Procédé selon la revendication 1, **caractérisé en ce que** les poussières inertes sont choisies dans le groupe consistant en : des farines de bois, des résidus d'olive épuisés, des carburants sous la forme de fines particules, un carbonate de métaux alcalins ou alcalino-terreux, des cendres résiduelles de combustion, de l'argile et de la terre de diatomées.

3. Procédé selon la revendication 1, **caractérisé en ce que** les particules solides sont dispersées dans le gaz dans le même sens que le, ou dans le sens opposé au, courant ou sont entraînées dans l'écoulement de gaz par l'épuration du gaz dans le lit de poussières.

4. Procédé selon la revendication 1, **caractérisé en ce que** la dispersion de poussières inertes est obtenue par des mélangeurs à écoulement statique avec un haut rendement.

5. Procédé selon la revendication 1, **caractérisé en ce que** les particules solides peuvent être séparées du gaz en tant que masses solides sous la forme de poussière sèche par des systèmes d'épuisement de poussière connus tels que des cyclones et/ou des filtres à poussière et/ou des filtres électrostatiques.

6. Procédé selon la revendication 1, **caractérisé en ce qu'**après avoir séparé le milieu polluant du gaz, les particules solides sont thermiquement régénérées pour être de nouveau utilisées dans un cycle de purification suivant, par grillage dans un four de régénération.

7. Procédé selon la revendication précédente, **caractérisé en ce qu'**il est prévu des dispositifs capables d'assurer l'oxydation des fractions volatiles qui se dégagent du four où la régénération a lieu de façon à assurer la dégradation complète avant qu'elles ne rejoignent l'atmosphère.

8. Procédé selon la revendication précédente, **caractérisé en ce que** lesdits dispositifs sont typiquement des chalumeaux de Bunsen alimentés par un carburant gazeux et pourvus d'un excès d'air et de moyens de régulation de température et d'une aide catalytique à la combustion.
